# EUROPÄISCHE PATENTSCHRIFT

(11) **EP 1 634 577 B1**
(45) Veröffentlichungstag und Bekanntmachung des Hinweises auf die Patenterteilung: **23.04.2008**
(21) Anmeldenummer: 05017722.9
(22) Anmeldetag: 16.08.2005
(51) Int. Cl.: A61K 8/66, A61Q 5/06, A61Q 5/10

(54) **Verfahren zur enzymatischen Vorbehandlung von färbenden keratinischen Fasern**
Process for the enzymatic pre-colouration treatment of keratin fibres
Procédé enzymatique pré-traitante pour la coloration des fibres kératiniques

(30) Priorität: 26.08.2004 DE 102004041570
(43) Veröffentlichungstag der Anmeldung: 15.03.2006
(73) Patentinhaber: Henkel Kommanditgesellschaft auf Aktien, 40589 Düsseldorf (DE)
(72) Erfinder: Otto, Ralf, 88422 Oggelshausen (DE); Kleen, Astrid, 22763 Hamburg (DE); Meinigke, Bernd, 51381 Leverkusen (DE); Rose, David, 40723 Hilden (DE); Sättler, Andrea, 40225 Düsseldorf (DE); Höffkes, Horst, 40595 Düsseldorf (DE); Naumann, Frank, 40219 Düsseldorf (DE)

(56) Entgegenhaltungen:
- EP-A- 0 530 865
- WO-A-99/00489
- US-A- 4 556 554
- US-A- 5 738 879
- PATENT ABSTRACTS OF JAPAN Bd. 012, Nr. 382 (C-535), 12. Oktober 1988 (1988-10-12) & JP 63 130514 A (SHISEIDO CO LTD), 2. Juni 1988 (1988-06-02)

## Beschreibung

Die vorliegende Erfindung betrifft ein Mittel zur enzymatischen Vorbehandlung zu färbender keratinischer Fasern, entsprechende Verwendungen sowie Verfahren zur Vorbehandlung von Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln. Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fallen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schwelßechlheit. Waschechtheit etc.) auf, so kann es mit der Zeit zu einer erkennbaren und daher unerwünschten Farbverschiebung kommen. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Schließlich hat in jüngerer Zeit ein weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoff. vorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muß. Bei Personen mit unsprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat nicht an Anstrengungen gefehlt, die Haltbarkeit und die Farbintensität von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Haltbarkeit und die Farbintensität der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird.

Es wurde nun überraschenderweise gefunden, dass durch eine dem Färbevorgang vorangehende enzymatische Vorbehandlung die Haltbarkeit und die Farbintensität von Färbungen keratinischer Fasern signifikant gesteigert werden können.

Gegenstand der vorliegenden Erfindung ist daher ein Verfahren zur Färbung oder Tönung keratinischer Fasern, wobei man In einem ersten Schritt eine Vorbehandlung mit einem Mittel, enthaltend mindestens ein Enzym, das ausgewählt ist unter Carboxyesterhydrolasen und Proteasen, vornimmt und in einem zweiten Schritt das Färbe- oder Tönungsmittel aufbringt.

Unter Haltbarkeit im Sinne der vorliegenden Erfindung ist die Erhaltung der ursprünglichen Färbung hinsichtlich Nuance und/oder Intensität zu verstehen, wenn die keratinische Faser dem wiederholten Einfluß von Licht oder von wäßrigen Mitteln, insbesondere tensidhaltigen Mitteln wie Shampoos, ausgesetzt wird.

Unter enzymatischer Vorbehandlung versteht man erfindungsgemäß Enzymkatalysierte Reaktionen an den Fasern, insbesondere Haarfasem, bei denen Haar-Poren vergrößert, oberste Haarschichten entfettet oder reaktive Seitengruppen der Aminosäuren des Haarkeratins freigelegt werden.

Unter keratinischen Fasern werden erfindungsgemäß Pelze, Wolle, Federn und insbesondere menschliche Haare verstanden.

Die Färbungen auf derartig vorbehandelten und anschließend gefärbten Keratinfasern (insbesondere auf menschlichen Haaren) zeigen besonders gute Haltbarkeit und erhöhte Farbintensität. Nach der Vorbehandlung können etablierte Färbemethoden oder neueste Entwicklungen wie z.B. enzymatische Haarfärbungsprozeduren angewendet werden.

Die enzymatische Vorbehandlung verbessert die Haltbarkeit der Färbung und die Farbintensität der Haare. Im Vergleich zur Vorbehandlung mit Reduktionsmitteln nach dem Stand der Technik zeigen die Haare nach Enzymeinwirkung besseren Griff und verkleben nicht.

Das im Rahmen des erfindungsgemäßen Verfahrens eingesetzte Mittel zur enzymatischen Vorbehandlung zu färbender keratinischer Fasern kann Kombinationen unterschiedlicher Enzyme enthalten; vorzugsweise mehrere voneinander verschiedene Cerboxylesterhydrolasen oder mehrere voneinander verschiedene Proteasen, aber auch Kombinationen von Carboxylesterhydrolasen und Proteasen.

Carboxylesterhydrolasen können die OberflöChenmembran der Faser einschließlich A-layer, der äussersten Proteinschicht der Haar-schuppenschicht, beeinflussen bzw. zu einer "Entfettung" beitragen.

In dem Vorbenhandlungsmittel grundsätzlich alle Carboxylestertlydrolasen einsetzbar, insbesondere die unter *E.C.3.1.1,-Carbaxylic ester hydrolases klassifizierter* Enzyme, die beispielsweise im Internet unter der URL http://www.biochem.ucl.ac.uk/bsm/enzymes/ec3/ec01/ec01/index.html aufgelistet sind.

Bevorzugte Carboxylesterhydrolasen sind erfindungsgemäß die Lipasen (*E.C.3.1.1.3 Triacylglycerol lipase*) und die Sterol-Esterasen (EC 3.1.1.13), die z. B. unter der URL http://www.biochem.uci.ac.uk/bsm/enzymes/ec3/ec01/ec01/ec0003/index.htm und http://ce.expasy.org/enzyme/ zu finden sind.. Industriell erhältliche, bzw. für technische Prozesse eingesetzte Lipasen stammen gegenwärtig beispielsweise aus den Pilzen Humicola lanuginosa, Candida cylindracea, Rhizopus arrhizus, Aspergillus niger und Geotrichum candidum, aus Bakterien der Gattung Pseudomonas spec. und Chromobacterium viscosum sowie aus Schweinepankreas und Weizenkeimgewebe. Die wichtigsten biotechnologisch eingesetzten Lipasebildner sind Mikroorganismen. Sowohl bei den Pilzen wie bei den Bakterien wurden einige Arten intensiver untersucht:

Lipasebildner unter den Pilzen:
Es gibt mehr als 300 Pilzspecies, von denen bekannt ist, dass sie Lipasen bilden. Von besonderem Interesse sind erfindungsgemäß Pilze aus der Familie der Mucoraceae, vor allem der Gattungen (Rhizo)Mucor und Rhizopus. Sie gehören zu den Zygomyceten (Jochpilzen), landbewohnenden niederen Pilzen mit unseptierten Hyphen, die bei Sauerstoffmangel ein hefeartiges Wachstum aufweisen. Ihre Zellwand enthält Chitin. In diese Gruppe gehören bekannte Arten wie der gemeine Brotschimmel Rhizopus nigricans und der gemeine Köpfchenschimmel Mucor mucedo. Das vegetative Mycel ist haploid.
Lipasegene der Gattung Mucor gibt es sowohl konstitutiv als auch induzierbar. Das Molekulargewicht der Mueor-Lipasen liegt um 30 kDa. sie zeigen maximale Aktivität bei Fetlsäurekettenlängen zwischen C8 und C12. Das Temperaturoptimum der Mucor-Llpasen liegt generell um 38°C.
Die Lipasen der Gattung Rhizopus zeigen 1.3-Reglospezifität und eignen sich dadurch für verschiedene Industrielle Anwendungen. Sie haben ein Temperaturoptimum zwischen 30 und 40°C. Die Lipase von R. delemar weist beispielsweise ein Temperaturoptimum von 30°C auf. Sie zeigen maximale Aktivität bei Fettsäurekottenfängen zwischen C8 und C10. Maximale lipolytische Aktivität wurde nach fünf Tagen bei pH 6 gefunden. Das Molekulargewicht der Rhizopus-Lipasen liegt im Bereich von 40 bis 45 kDa.

Lipase-bildende Bakterien:
Auch bei den Bakterien gibt es zahlreiche Lipasebildner. Genauer bekannt sind die Lipasen der Gattungen Pseudomonas, Bacillus und Staphylococcus. Molekulargenetische Untersuchungen wurden vor allem an Pseudomonas-Lipasen durchgeführt. An Gram-positiven Organismen wurden Staphylococcus- und Bacillus-Arten untersucht. Die Lipasen Gram-negativer und Gram-positiver Stämme unterscheiden sich deutlich voneinander.

Lipasen Gram-negativer Bakterien:
Das durchschnittliche Molekulargewicht von Pseudomonas-Lipasen liegt bei 30 kDa. Die Homologie untereinander ist meist größer als 60%. Es gibt aber auch Ps.-Lipasen, die nur eine deutlich geringere Homologie untereinander aufweisen. Es gibt hier, wie auch bei allen anderen Lipasen, eine hochkonservierte Sequenz, ein Pentapeptid (G-X-S-X-G). Dabei handelt es sich um einen Teil des aktiven Zentrums, das in der gesamten Gruppe der Serin-Hydrolasen, zu der auch die Lipasen gerechnet werden, gefunden werden kann. Diese Serin-Hydrolasen bilden eine Gruppe von Enzymen mit ähnlicher Struktur, dem sogenannten "hydrolase-fold" sowie einem ähnlichem Reaktionsmechanismus.

Lipasen Gram-positiver Bakterien:
Hier gibt es zwischen den einzelnen Gattungen deutliche Unterschiede.
Das aktive, reife Protein der Gattung Staphylococcus ist 44-48 kDa groß, die Sequenzhomologie der Lipasen der einzelnen Slämme Im sogenannten Präpro-Teil betragt nur 25 %, die reifen Enzyme zeigen dagegen 65 %Ige Homologie untereinander. Bis auf das aktive Zentrum und eine Region im N-terminalen Bereich zeigen sich keine nennenswerten Homologien mit den Pseudomones-Lipasen.
Die Lipasen der Gattung Bacillus sind mit 20 - 22 kDa deutlich kleiner als die übrigen Lipasen, die Molekulargewichte von 40-50 kDa haben. Auch in der konservierten Sequenz (G-X-S-X-G) des aktiven Zentrums gibt es Unterschiede: An Position 1 findet sich ein Alanin statt des sonst üblichen Glycins. Eine derartige katalytische Sequenz findet man auch bei der Protease Subtilisin.

Besonders bevorzugt sind erfindungsgemäß Upasen, die mittels dem Fachmann bekannter Methoden aus folgenden Organismen bzw. Geweben erhältlich sind: Candida antartica. Candida rugosa; Pseudomonas species; Schweinepankreas; Thermomyces lanuginosa; Mucor mihei; Alcallgenes species.

Sterolesterasen können aus Rattenleber oder -pankreas oder auch rekombinant aus Bakterien oder Pilzen gewonnen werden Besonders bevorzugt sind jedoch aus Schweineleber gewonnene Sterol-Eeterasen sowie aus Schweinepankreas gewonnene Sterol-Esterasen.

Proteasen spalten Peptid-Bindungen innerhalb einer Aminosäure-Kette. Dadurch kann die Abschuppung verbessert, Poren können vergrößert und Aminosäure-Seitenketten können freigelegt werden. Somit kann das Eindringen der Farbstoff-Moleküle ins Haar oder im Falle direktkopplender Farbstoffmoleküle deren Reaktion mit freien Gruppen Innerhalb von Proteinen verbessert werden.

Es können erfindungsgemäß pro- und/oder eukaryotische Proteasen eingesetzt werden. Sie unterscheiden sich unter anderem durch ihre Spaltspezifität (gezielt nach bestimmten Aminosäuren in einer Kette oder zufällig). Beispielsweise spaltet Trypsin Peptid-Blndungen nach den Aminosäuren Arginin und Lysin. Dabei entstehen freie Amino-Gruppen an den Protein-Seitenketten, mit denen entsprechende Reaktionen möglich sind. Die Keratin-Struktur wird weniger stark angegriffen als bei dem Einsatz von unspezlfisch spaltenden Proteasen wie z.B. bakterieller Subtilisine.

In dem Vorbehandlungsmittel grundsätzlich alle Proteasen einsetzbar, insbesondere die unter EC 3.4
- *Acting on peptide bonds (Peptidases)* klassifizierten Enzyme, die beispielsweise im Internet unter der URL
   http://www.chem.qmul.ac.uk/lubmb/enzyme/EC3/4/
   und http://ca.expasy.org/cgi-bin/enzyme-search-cl aufgelistet sind.

Bevorzugte Proteasen sind erfindungsgemäß die Serin Endopeptidasen *(E.C.3.4.21.x)* und die Metalloendopeptldasen (*E.C.3.4.24.x*).

Erfindungsgemäß besonders bevorzugte Proteasen sind ausgewählt unter Alkalase^{®} *(EC* 3.4.21.62, z. B. erhältlich von der Fa. Novozymes) und BLAP (Henkel-Protease aus Bacillus spec.), die beide zu den Subtillsinen gehören sowie Pronase E® (Streptomyces griseus neutral proteinase, EC *3.4.24.31.*

Das Vorbehandlungsmittel kann zusätzlich ein Reduktionsmittel, wie z. B. Dithiothreitol, Thioglykolsäure oder Natriumsulfid/Natyriumsulflt, enthalten. Solche Reduktionsmittel reduzieren die Disuifid-Brücken der starren Haarfaserstruktur. Mit den freien SH-Gruppen innerhalb der Aminosäure-Ketten des alpha-Keratins können z. B. kovalent koppelnde Farbstoff-Moiekole reagieren.

Das Vorbehandlungsmittel ist besonders geeignet, um Haare für eine nachfolgende enzymatisch oxidative Färbung, insbesondere mit Laccasen, Tyrosinasen, Oricasen bzw. Cholinoxidasen/Peroxidasen vorzubehandein.

Die Enzyme sind in dem Vorbehandlungsmittel bevorzugt in Mengen von 0,005 bis 5 Gew.-%, bezogen auf die gesamte Zubereitung, enthalten. Mengen von 0,05 bis 2 Gew.-% sind besonders bevorzugt, Mengen von 0.01 - 0.1 Gew-% sind ganz besonders bevorzugt. Verfahren

Hinsichtlich des zeltlichen Ablafus unterlieg das erfindungsgemäße Verfahren keinerlei Beschrankungen. Es sollte allerdings zwischen der Vorbehandlung durch das erfindungsgemaße Mittel und der nachfolgenden Färbung kein allzu großer zeitlicher Abstand liegen, so dass die Fasern zwischen den Schritten nicht trocknen, bzw. eine Rückbildung der erzieiten Vorbeheindlungsegekte nicht erfolgen kann

Obwohl das Vorbehandlungsmittel prinzipiell auf dem Haar verbleiben kann, und z.B. mit dem anschliessend aufgetragenen Färbemittel ausgespült werden könnte, wird das Mittel vorzugsweise nach einer Einwirkzeit von 1 Minute bis 60 Minuten ausgespült. Dieses Ausspülen kann mit reinem Wasser oder einem marktüblichen Shampoo erfolgen. Einwirkzeiten von 2 bis 30 Minuten, insbesondere von 2 bis 15 Minuten haben sich in den meisten Fällen als besonders geeignet erwiesen. Unabhängig von dem genauen Ablauf der Vorbehandlung hat es sich als vorteilhaft erwiesen, das Vorbehandlungsmittel bei einer Temperatur von 20 bis 55 °C. Insbesondere von 25 bis 35°C, anzuwenden. Hinsichtlich der Art, gemäß der das Vorbehandlungsmittel auf die keratinische Faser, insbesondere das menschliche Haar, aufgebracht wird, bestehen keine prinzipiellen Einschränkungen. Als Konfektionierung dieser das das erfindungsgemäß Mittel enthaltenden Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Diese werden in der Regel auf wäßriger oder wäßrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können In der wäßrig alkoholischen Basis in einem Gewichtsverhältnis von 1 : 10 bis 10 : 1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

Neben dem erfindungsgemäß zwingend erforderlichen Enzym und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionogene Tenside wie beispielsweise Alkylphenolpolyglycolether, Feltsäurepolyglycolester, Fettsäureamldpolyglycolether, Fetteminpolyglycolether, alkoxylierte Triglyceride, wie insbesondere ethoxyliertes Rizinusöl, Alk(en) yloligoglucoside, Fettsäure-N-alkylglucsmide, Polyolfettsäureester, Zuckerester, Sorbllanester und Polysorbats. Sofern die nichtionischen Tenside Polyglycoletherketten enthalten, können sie eine konventionelle oder eingeengte Homologenverteilung aufweisen.
- anionische Tenside, insbesondere Alkylsulfate, Alkylpolyglykolethersulfate und Ethercarbonsäuren mit 10 bis 18 C-Atomen in der Alkylgruppe und bis zu 12Glykolethergruppen im Molekül, Seifen sowie Sulfobemsteinsäuremono-und dialkylester mit 8 bis 18 C-Atomen in der Alkylgruppe und Sulfobernsteinsäuremono-alkylpolyoxyethyl-ester mit 8 bis 16 C-Alomen in der Alkylgruppe und 1 bis 6 Oxyethylgruppen,
- zwitterionische Tenside, insbesondere die sogenannten Betaine wie die N-Alkyl-N,N-dimethylammonlum-glycinate, beispielsweise das Kokosalkyl-dimethylammonium-glydnat, N-ACyl-aminopropyl-N,N-dimethylammoniumglycina-te, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.
- ampholytische Tenside wie beispielsweise N-Alkylglyclne, N-Alkylpropionsäuren, N-Alkylaminobuttersäuren. N-Alkyllminodiproplonsäuren, N-Hydroxyethyl-N-alkylamidopropylglycine, N-Alkyltaurine, N-Alkylssrcosine. 2-Alkylaminopropionsäuren und Alkyl-amlnoesslgsäuren mit jeweils etwa 8 bis 18 C-Atomen in der Alkylgruppe,
- nichtionische Polymere wie beispielsweise Vinylpyrrolldon/Vinylacrylat-Copoly mere, Polyvinylpyrrolidon und Vinylpyrrolidon/Vinylacotat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalacithin, Ei-Lecltin und Kephaline, sowie Silikonöle,
- Parfümöle, Dimethylisosorbid und Cyclodextrine.
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octyiether, Di-n-decylether, Di-n-nonylether, Di-n-undecylether und Di-n-dodecylether, n-Hexyl-n-oclylether, n-Octyl-n-decylether, n-Daoyl-n-undecylether, n-Undecyl-n-dodecylether und n-Hexyl-n-Undecylether sowie Dl-tert-butylether, Dl-iso-pentylether. Di-3-ethyldecylether. tert-Butyl-n-octytother, iso-Pentyl-n-octylether und 2-Methyl-pentyl-n-octylether,
- FaltalKohote, insbesondere lineare und/oder gesättigte Fettalkohole mit 8 bis 30 C-Atomen, und Monoester der Fettsäuren mit Alkoholen mit 6 bis 24 C-Atomen,
- faserstrukturverbessernde Wirkstoffe, insbesondere Mono-, Di- und Oligosaccharide. wie beispielsweise Glucose, Galactose, Fructose, Fruchtzucher und Lactose,
- konditionierende Wirkstoffe wie Paraffinöle, pflanzliche Öle, z. B. Sonnenblumenöl, Orangenöl, Mandelöl. Weizenkeimöl und Pfirsichkernöl sowie Phospholipide, beispielsweise Sojalecithin, Ei-Lecithin und Kephaline,
- quaternierte Amine wie Methyl-1-alkylamidoethyl-2-alkylimidazollnlum-methosullat,
- Entschäumer wie Silikone,
- Farbstoffe zum Anfärben des Mittels,
- AntischuppenvArkstoffe wie Plroctone Olamine, Zink Omadine und Climbazol, LichtschuLzmittel, insbesondere derivatisierte Benzophenone, Zimtsäure-Derivate und Triazine.
- weitere Substanzen zur Einstellung des pH-Wertes, wie beispielsweise α- und β-Hydroxycarbonsäuren
- Wirkstoffe wie Allentoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide.
- Komplexbildner wie EDTA, NTA, β-Alamindiessigsäure und Phosphonsäuren,
- Quell- und Penetrationsstoffe wie Glycerin, Propyenglykolmonoethylelher, Carbonate, Hydrogencarbonate. Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomlichsäure, Cysteamin, Thioäpfelsäure und α-Mercaptoethansulfonsäure,
- Treibmittel wie Propan-Butan-Gemlsche, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Außage, Hüthig Buch Verlag, Heldelberg, 1989, verwiesen.

Ein weiterer Gegenstand der vorliegenden Erfindung Ist ein zweiteiliges Kit zur Färbung oder Tönung keratinischer Fasern. das dadurch gekennzeichnet ist, dass es
a) ein Mittel enthaltend mindestens ein Enzym, das ausgewählt ist unter Carboxylesterhydrolasen und Proteasen und
b) ein Färbe- oder Tönungsmittel
umfasst.

Die Zusammensetzung des einsetzbaren Färbe-oder Tönungsmittels unterliegt keinen prinziplellen Einschränkungen.
Als Farbstoff(vorprodukt)e können diese Mittel
- Oxidationsfarbstoffvorprodukte vom EntwiCkler- und Kuppler-Typ,
- natürliche und synthetische direktziehende Farbstoffe oder
- Vorstufen naturanaloger Farbstoffe, wie Indol- und Indolin-Derivate.
   sowie Mischungen von Vertretern einer oder mehrerer dieser Gruppen eingesetzt werden.

Es kann erfindungsgemäß bevorzugt sein, als EntwicKletkomponente ein p-Phenylendiaminderivat oder eines seiner physiologisch verträglichen Salze einzusetzen. Besonders bevorzugt sind p-Phenylendiaminderivate der Formel (E1) 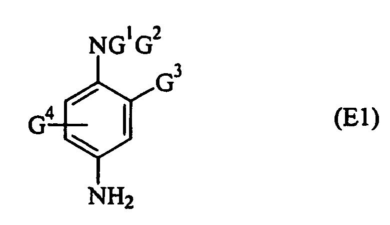 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁-bis C₄₋ Alkylrest, dermiteinerstickstoffhaltigen Gruppe, einem Phenyl-oder einem 4'-Aminophenylrestsubstituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄₋Monohydroxyalkylrest, einen C₂- bis C₄-Polyhydroxyalkylrest, einen C₁- bis C₄₋Hydroxyalkoxyrest, einen C₁- bis C₄-Acetylaminoalkoxyrest, einen C₁- bis C₄₋Mesylaminoalkoxyrest oder einen C₁-bis C₄-Carbamoylaminoalkoxyrest;
- G⁴ steht für ein Wasserstoffatom, ein Halogenatom oder einen C₁- bis C₄₋Alkylrest oder
- wenn G³ und G⁴ in ortho-Stellung zueinander stehen, können sie gemeinsam eine verbrückende α,ω-Alkylendio-xogruppe, wie beispielsweise eine Ethylendioxygruppe bilden.

Beispiele für die als Substituenten in den Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄₋Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄₋Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethylp-phenylendiamin, 2,5-Dimethyl-p-phenylendiamin, N,N-Dimethyl-p-phenylendiamin, N,N-Diethyl-p-phenylendiamin, N, N-Dipropyl-p-phenylendiamin, 4-Amino-3-methyl-(N,N-diethyl)-anilin, N,N-Bis-(β-hydroxyethyl)-p-phenylendiamin, 4-N, N-Bis-(β-hydroxyethyl)-amino-2-methylanilin, 4-N,N-Bis-(β-hydroxyethyl)-amino-2-chloranilin, 2-(β-Hydroxyethyl) -p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl) -p-phenylendiamin, N-Phenyl-p-phenylendiamin, 2-(β-Hydroxyethyloxy)-p-phenylendiamin, 2-(β-Acetylaminoethyloxy) -p-phenylendiamin, N-(β-Methoxyethyl)-p-phenylendiamin, N-(4-Amino-3-methylphenyl)-N-[3-(1H-imidazol-1-yl)propyl] amin und 5,8-Diaminobenzo-1,4-dioxan sowie ihren physiologisch verträglichen Salzen.

Ganz besonders bevorzugte p-Phenylendiaminderivate der Formel (E1) sind p-Phenylendiamin, p-Toluylendiamin, 2-(β-Hydroxyethyl)-p-phenylendiamin, 2-(a,β-Dihydroxyethyl)-p-phenylendiamin und N, N-Bis-(β-hydroxyethyl)-p-phenylendiamin.

Es kann weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 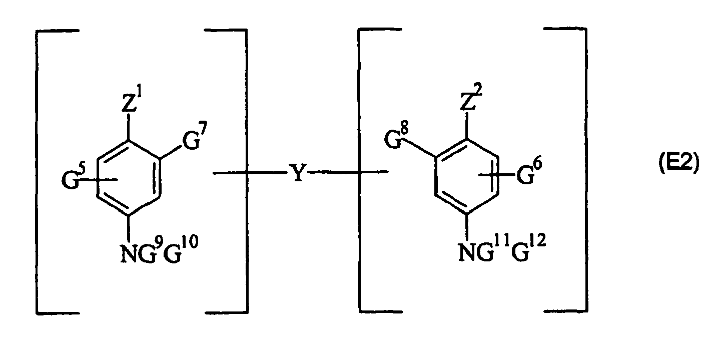 wobei:
- Z¹ und Z² stehen unabhängig voneinander für einen Hydroxyl- oder NH₂₋Rest, der gegebenenfalls durch einen C₁-bis C₄-Alkylrest, durch einen C₁- bis C₄₋Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/ oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄₋Alkylrest, mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl) -N,N'-bis-(4-aminophenyl)-1,3-diamino-propan-2-ol, N,N'-Bis-(β-hydroxyethyl)-N,N'-bis-(4-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4-amino-3-methylphenyl) -ethylendiamin, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, N,N'-Bis-(2-hydroxy-5-aminobenzyl)-piperazin, N-(4-Aminophenyl)-p-phenylendiamin und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diaminopropan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 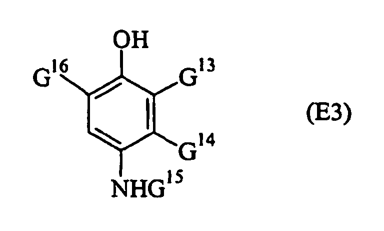 wobei:
- G¹³ steht für ein Wasserstoffatom, ein Halogenatom, einen C₁- bis C₄₋Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄₋Alkylrest, einen C₁- bis C₄-Monohydroxyalkylrest, einen C₂- bis C₄₋Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄₋Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 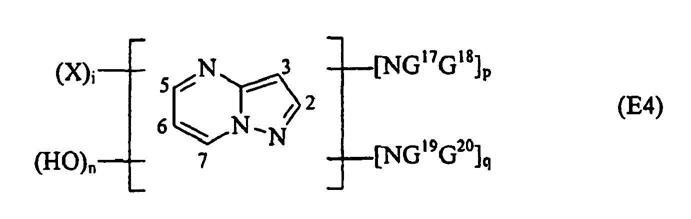 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄₋Hydroxyalkylrest, einen C₂- bis C₄-Polyhydroxyalkylrest einen (C₁- bis C₄)-Alkoxy-(C₁-bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁-bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄₋Hydroxyalkyl- oder einen Di-(C₁- bis C₄)-[Hydroxyalkyl]-(C₁-bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄- Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄₋Hydroxyalkyl- oder einen Di-(C₁- bis C₄-hydroxyalkyl)-aminoalkylrest, einen Aminorest, einen C₁-bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

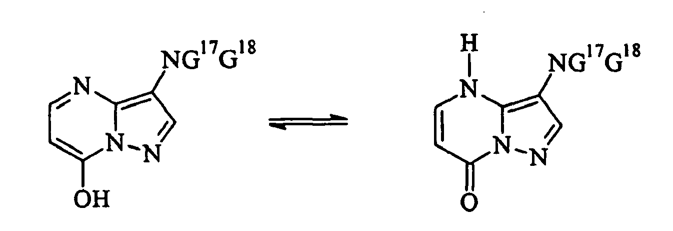

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1 ,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino) ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1 -Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diamlnopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die direktziehenden Farbstoffe sind vorzugsweise ausgewählt aus den Nitrophenylendiaminen, den Nitroaminophenolen, den Azofarbstoffen, den Anthrachinonen oder den Indophenolen. Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner kann es bevorzugt sein, dass die Mittel mindestens einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die mindestens einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

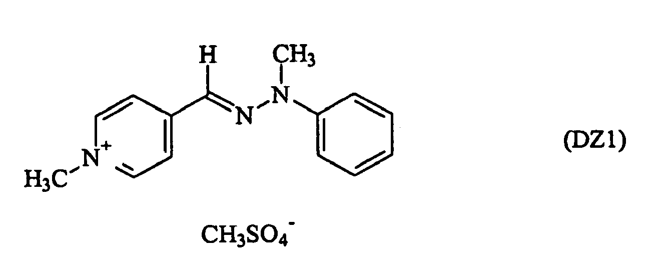

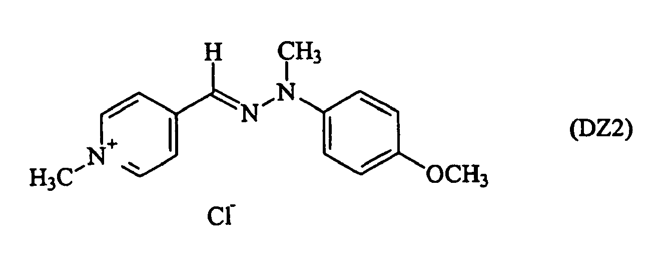

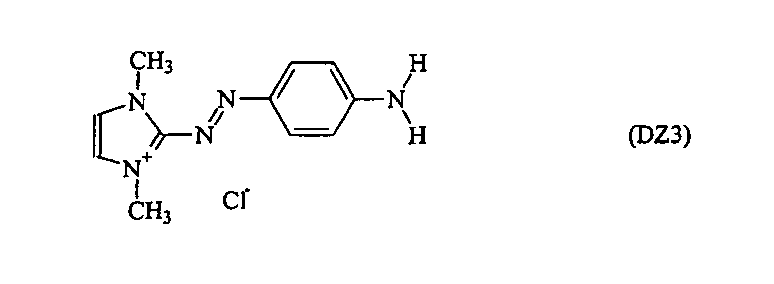

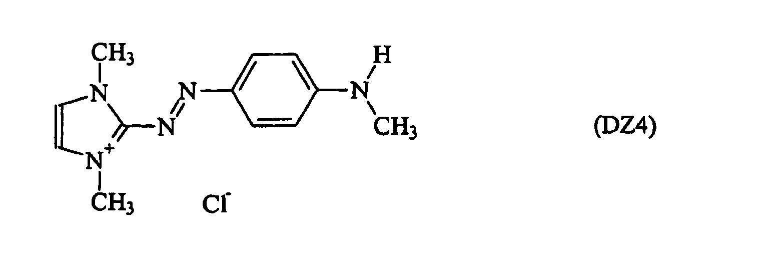

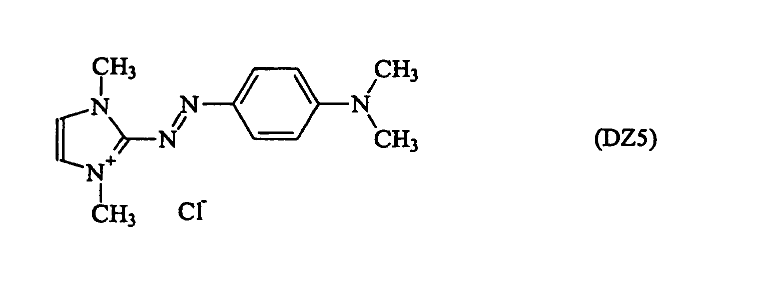

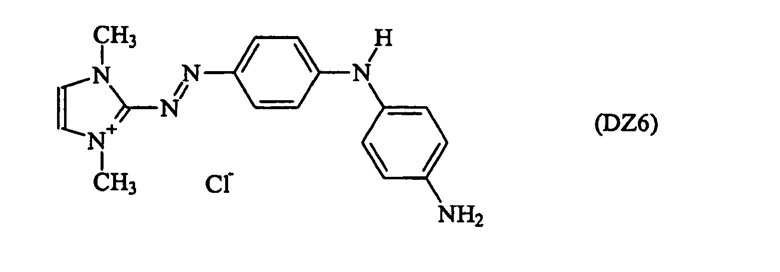

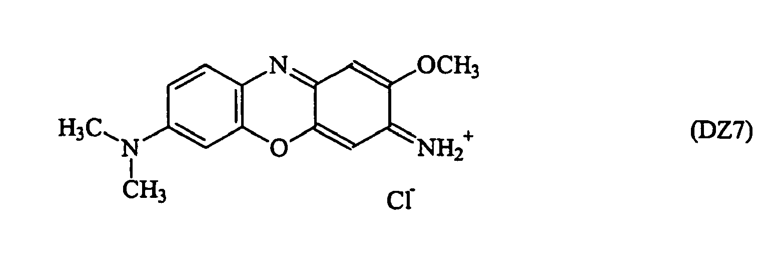

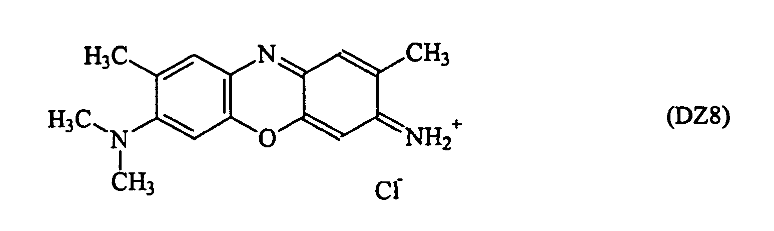

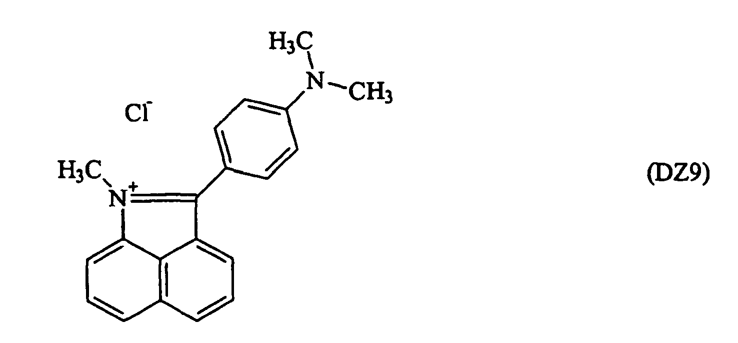

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Weiterhin können die Zubereitungen auch in der Natur vorkommende direktziehende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die Mittel enthalten die direktziehenden Farbstoffe bevorzugt in einer Menge von 0,01 bis 20 Gew.-%, bezogen auf die gesamten Anwendungszubereitung.

Es ist nicht erforderlich, dass die Oxidationsfarbstoffvorprodukte oder die direktziehenden Farbstoffe jeweils einheitliche Verbindungen darstellen. Vielmehr können in den Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden bevorzugt Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer bevorzugten Ausführungsform enthalten die Zweikomponentenmittel daher mindestens ein Indol-und/oder ein Indolinderivat.

Besonders gut als Vorstufen naturanaloger Haarfarbstoffe geeignet sind Derivate des 5,6-Dihydroxyindolins der Formel (la), 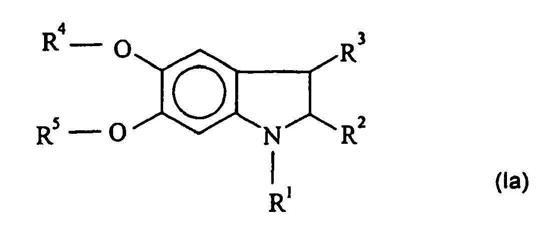 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ stehtfür eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Beispiele für die ais Substituenten in den Verbindungen genannten C₁-bis C₄-Aikyigruppen sind die Gruppen Methyl, Ethyl, Propyl, Isopropyl und Butyl. Ethyl und Methyl sind bevorzugte Alkylgruppen. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 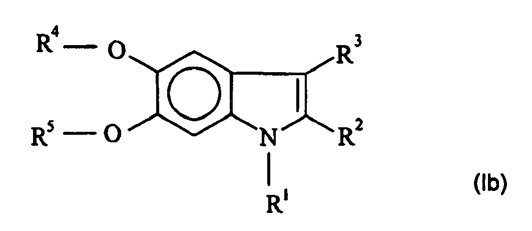 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ stehtfür eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Beispiele für die als Substituenten in den Verbindungen genannten C₁-bis C₄-Alkylgruppen sind die Gruppen Methyl, Ethyl, Propyl, Isopropyl und Butyl. Ethyl und Methyl sind bevorzugte Alkylgruppen. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Mitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-%, jeweils bezogen auf die gesamte Anwendungszubereitung enthalten.

Es kann bevorzugt sein, das Indolin- oder Indolderivat in den Zweikomponentenmitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin. Dabei spielt es erfindungsgemäß keine Rolle, ob die Aminosäure gemeinsam mit dem Indolin-oder Indolderivat in der Zubereitung (A) enthalten ist oder ob die Aminosäure getrennt von dem Indolin- oder Indolderivat in der Zubereitung (B) enthalten ist.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li+, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwerfärbbarem Haar kann die Zubereitung mit den Farbstoffvorprodukten aber auch ohne vorherige Vermischung mit derOxidationskomponente auf das Haar aufgebracht werden. Nach einer Einwirkdauervon 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

### Ausführungsbeispiele

Die folgenden Beispiele erläutern die Erfindung, ohne sie jedoch darauf einzuschränken:

Die erhaltenen Farbtöne und Intensitäten wurden anhand von Farbtafeln (Taschenlexikon der Farben, A. Kornerup u. J. H. Wanscher, Muster-Schmidt-Verlag, 3. unveränderte Aufl. 1981) bestimmt. Des weiteren wurden auch Messungen nach dem Dreibereichsverfahren nach DIN 5033 durchgeführt.

### Beispiel 1: Semipermanente Haarfärbung nach Vorbehandlung der Haare:

Durch die semipermanente Haarfärbung sollen dauerhafte Nuancen, die 5 bis 6 Haarwäschen halten, erreicht werden. Die Haare wurden mit Proteasen, und/oder Reduktionsmitteln vorbehandelt und anschließend semipermanent, mit dem Handelsprodukt Poly Live® Soft Töner, gefärbt. Die Messung der Farbintensität erfolgte farbmetrisch im Vergleich zu einer unbehandelten Haarsträhne.

Die Ergebnisse sind im folgenden Diagramm graphisch dargestellt. 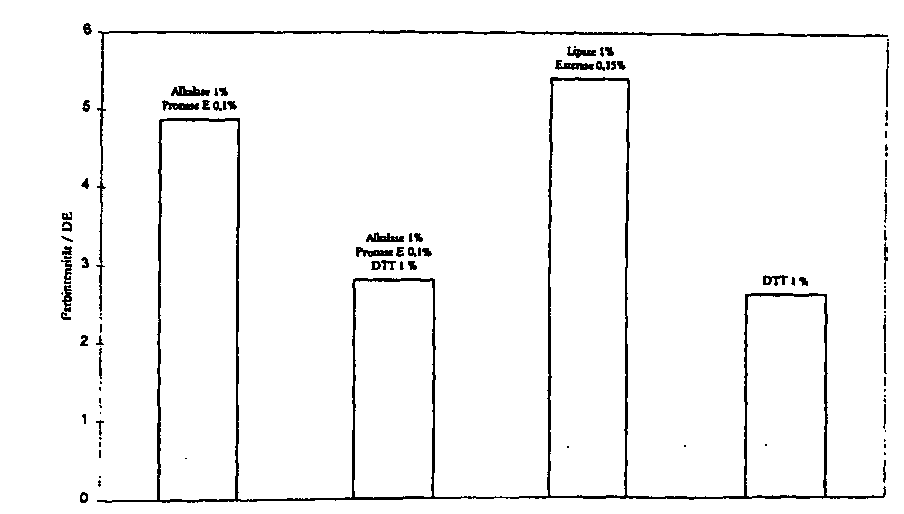 Diagramm 4.11: Farbintensität nach Haar-Vorschädigung und anschließender semipermanenter Färbung

Die enzymatische Vorbehandlung mit Esterhydrolasen (Lipase und Esterase) oder einer Protasekombination erbrachte die besten Effekte. Beispielsweise wurde die Lipase aus Candida antarctica mit 0,5 und 1 Gew.-% in einer Basiscreme auf menschliche Haarproben angewendet und anschliessend die Färbung durchgeführt. Die so behandelten Haarsträhnen zeigten keine optische Schädigung.
Die mit Dithiothreitol (DTT) vorbehandelten Haarsträhnen zeigten hingegen eine starke optische Schädigung. Die Haare waren brüchig, rauh und schwer kämmbar.

### Beispiel 2: Permanente oxidative Haarfärbung nach Vorbehandlung der Haare:

Die Haar-Strähnen wurden mit Proteasen, Lipasen optional mit Reduktionsmitteln vorbehandelt und anschlie-ßend permanent enzymatisch-oxidativ mit Tyrosinase bzw. Cholinoxidase/Peroxidase gefärbt. Die Farbintensitäten bzw. Farbzahlen (Komerup, A., Wanscher, J. H., 1981) sind in Tabelle 1 dargestellt. Es wurde eine leichte Verbesserung festgestellt.

**Tabelle 1: Farbintensitäten der enzymatisch vorbehandelten und permanent gefärbten Haarsträhnen**

| | permanente Färbung mit: | | | |
|---|---|---|---|---|
| | *Tyrosinase* | | *Cholinoxidase*/*Peroxidase* | |
| Vorbehandlung mit: | Farbzahl - | Farbe | Farbzahl | Farbe |
| Alkalase/Pronase E/DIT | 5 E6¹⁾ | senf-braun¹⁾ | 6 F8 | dunkel-braun |
| Trypsin | 5 E3 | maus-grau | 5E6 | senf-braun |
| Trypsin/DTI | 5 E4 | haar-braun | 5 F8 | umbra |
| Lipase (A)/Lipase (B) | 5 E4: | haar-braun | 5 E6 | Senf-braun |
| unbehandelt | 5 E4 | haar-braun | 5 F7 | kaffee-braun |

| | | | | |
|---|---|---|---|---|
| ¹⁾ ohne Pronase E; | | | | |

je höher die Farbzahl, um so stärker ist die Farbintensität. Jede Farbzahl steht für eine entsprechende Farbe bzw. Grundfarbe welch im Taschenlexikon der Farben (Komerup, A., Wanscher, JH., 1981) beschrieben ist.

### Beispiel 3: Permanente Haarfärbung durch Farbstoffe, die chemisch an das Haar gebunden werden, nach Vorbehandlung der Haare:

Es wurde die Vorbehandlung mit Enzymen wie Protease bzw. Lipase untersucht. Die Auswertung erfolgte optisch sowie durch die Bestimmung der Farbintensität mit Hilfe einer farbmetrischen Tabelle.
Die Farbintensitäten nach der Vorbehandlung mit Enzymen sind in Diagramm 4.12 graphisch dargestellt. 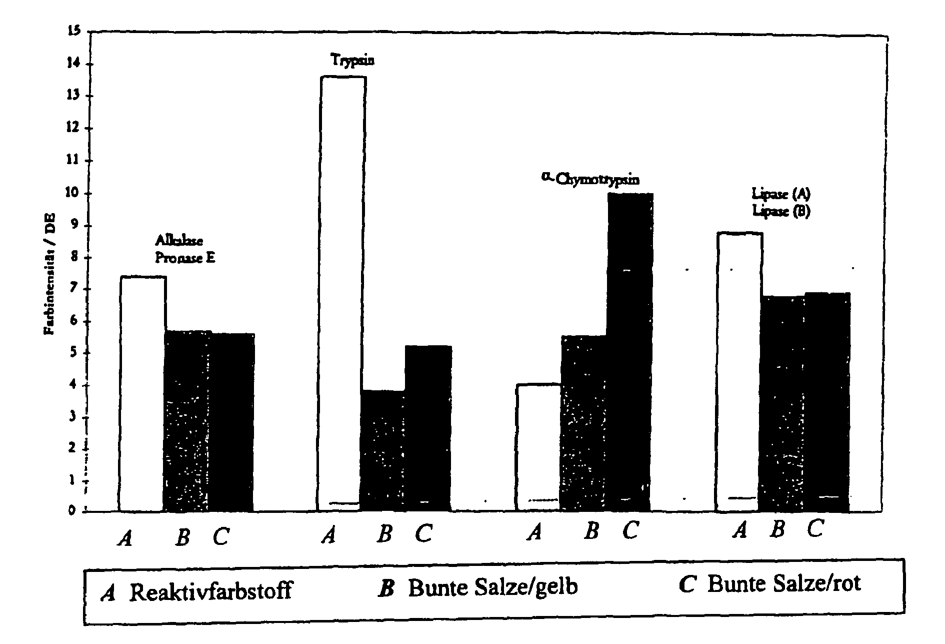 Diagramm 4.12: Farbintensität nach enzymatischer Vorberhandlung von Haaren, mit anschließender Färbung durch Farbstoffe, die chemisch an das Haar gebunden werden.

Alle Variationen der enzymatischen Vorbehandlung verbesserten die Farbintensität der Haare.

Im Vergleich zur Behandlung mit Reduktionsmitteln zeigten die Haare nach Enzymeinwirkung besseren Griff und verklebten nicht.

## Patentansprüche

1. Verfahren zur Färbung oder Tönung keratinischer Fasern, **dadurch gekennzeichnet, dass** man in einem ersten Schritt eine Vorbehandlung mit einem Mittel, enthaltend mindestens ein Enzym, das ausgewählt ist unter Carboxylesterhydrolasen und Proteasen, vornimmt und in einem zweiten Schritt das Farbe- oder Tönungsmittel aufbringt.

2. Verfahren nach Anspruch 1, **dadurch gekennzeichnet, dass** das Mittel Kombinationen unterschiedlicher Enzyme enthält; vorzugsweise mehrere voneinander verschiedene Carboxylesterhydrolasen oder mehrere voneinander verschiedene Proteasen, oder Kombinationen von Carboxylesterhydrolasen und Proteasen.

3. Verfahren nach Anspruch 1 oder 2, **dadurch gekennzeichnet, dass** das Mittel Carboxylesterhydrolasen enthält, die ausgewählt sind unter Lipasen und Sterolesterasen, insbesondere unter Lipasen, die aus Candida antartica, Candida rugosa; Pseudomonas species; Schweinepankreas; Thermomyces lanuginosa; Mucor mihei und Alcaligenes species erhältlich sind sowie unter Sterolesterasen, die aus Schweineleber oder Schweinepankreas erhältlich sind.

4. Verfahren nach einem der vorhergehenden Ansprüche, **dadurch gekennzeichnet, dass** das Mittel die Enzyme in Mengen von 0,005 bis 6 Gew.-%, insbesondere von 0,05 bis 2 Gew.-%, vorzugsweise von 0.01 - 0.1 Gew-%, bezogen auf die gesamte Zubereitung, enthält.

5. Zweiteiliges Kit zur Färbung oder Tönung keratinischer Fasern, **dadurch gekennzeichnet, dass** es
a) ein Mittel, enthaltend mindestens ein Enzym, das ausgewählt ist unter Carboxylesterhydrolasen und Proteasen, und
b) ein Färbe oder Tönungsmittel umfasst.

## Claims

1. Process for the colouring or tinting of keratin fibres, **characterized in that**, in a first step, a pretreatment is undertaken with a composition comprising at least one enzyme selected from carboxylic ester hydrolases and proteases, and, in a second step, the colourant or tint is applied.

2. Process according to Claim 1, **characterized in that** the composition comprises combinations of different enzymes; preferably a plurality of carboxylic ester hydrolases different from one another or a plurality of proteases different from one another, or combinations of carboxylic ester hydrolases and proteases.

3. Process according to Claim 1 or 2, **characterized in that** the composition comprises carboxylic ester hydrolases selected from lipases and sterol esterases, in particular from lipases obtainable from Candida antartica, Candida rugosa; Pseudomonas species; porcine pancreas; Thermomyces lanuginosa; Mucor mihei and Alcaligenes species, and also from sterol esterases obtainable from porcine liver or porcine pancreas.

4. Process according to one of the preceding claims, **characterized in that** the composition comprises the enzymes in amounts of from 0.005 to 5% by weight, in particular from 0.05 to 2% by weight, preferably from 0.01 - 0.1% by weight, based on the total preparation.

5. Two-part kit for colouring or tinting keratin fibres, **characterized in that** it comprises
a) a composition comprising at least one enzyme selected from carboxylic ester hydrolases and proteases, and
b) a colourant or tint.

## Revendications

1. Procédé pour teindre ou nuancer les fibres kératiniques, **caractérisé en ce que** dans une première étape, l'on procède à un prétraitement avec un agent contenant au moins une enzyme, qui est choisie parmi les carboxylester hydrolases et protéases et dans une deuxième étape l'on applique l'agent de teinture ou de nuançage.

2. Procédé selon la revendication 1, **caractérisé en ce que** l'agent contient des combinaisons d'enzymes différentes ; de préférence plusieurs carboxylester hydrolases différentes les unes des autres, ou plusieurs protéases différentes les unes des autres, ou des combinaisons de carboxylester hydrolases et de protéases.

3. Procédé selon la revendication 1 ou 2, **caractérisé en ce que** l'agent contient des carboxylester hydrolases qui sont choisies parmi les lipases et les stérol estérases, en particulier parmi les lipases que l'on peut obtenir à partir de *Candida antartica, Candida rugosa; Pseudomonas species;* de pancréas de porc; de *Thermomyces lanuginosa; Mucor mihei* et *Alcaligenes species,* ainsi que parmi les stérol estérases que l'on peut obtenir à partir de foie de porc ou de pancréas de porc.

4. Procédé selon l'une quelconque des revendications précédentes, **caractérisé en ce que** l'agent contient les enzymes en des quantités de 0,005 à 5% en poids, en particulier de 0,05 à 2% en poids, de préférence de 0,01 à 0, 1 % en poids, par rapport à la préparation totale.

5. Nécessaire en deux parties pour teindre ou nuancer les fibres kératiniques, **caractérisé en ce qu'**il comprend
a) un agent, contenant au moins une enzyme qui est choisie parmi les carboxylester hydrolases et les protéases, et
b) un agent de teinture ou de nuançage.
